# EUROPÄISCHE PATENTSCHRIFT

(11) **EP 0 488 059 B1**
(45) Veröffentlichungstag und Bekanntmachung des Hinweises auf die Patenterteilung: **06.09.1995**
(21) Anmeldenummer: 91119892.7
(22) Anmeldetag: 21.11.1991
(51) Int. Cl.: A61K 38/55, A61K 45/06

(54) **Verwendung einer Kombination aus Angiotensin-Converting-Enzyme-Hemmer und Calciumantagonist zur Behandlung einer Proteinurie**
Use of a combination of an ace inhibitor with a calcium antagonist in the treatment of proteinuria
Utilisation d'une association d'un inhibiteur ace avec un antagoniste du calcium pour traiter la protéinurie

(30) Priorität: 27.11.1990 DE 4037691
(43) Veröffentlichungstag der Anmeldung: 03.06.1992
(60) Teilanmeldung: 94117179.5
(73) Patentinhaber: HOECHST AKTIENGESELLSCHAFT, 65926 Frankfurt am Main (DE)
(72) Erfinder: Becker, Reinhard, Dr., W-6200 Wiesbaden (DE); Henning, Rainer, Dr., W-6234 Hattersheim am Main (DE); Teetz, Volker, Dr., W-6238 Hofheim am Taunus (DE); Urbach, Hansjörg, Dr., W-6242 Kronberg/Taunus (DE)

(56) Entgegenhaltungen:
- EP-A- 0 381 075
- J. CARDIOVASC. PHARMACOL. Bd. 16, Nr. 6, Dezember 1990, Seiten 924 - 930; H.J.KLOKE ET AL.: 'Effects of nitrendipine and cliazapril on renal hemodynamics andalbuminuria in hypertensive patients with chronic renal failure'
- Z. ALLGEMEINMED. Bd. 66, Nr. 32, 1990, Seite 924; A. FEICHTNER: 'ACE-Hemmerlindern die Proteinurie'
- J. CARDIOVASC. PHARMACOL. Bd. 10, Nr. S10, 1987, Seiten 167 - 169; B. JACKSONET AL.: 'Disparate effects of ACE-inhibitors and calcium-blocker treatment onthe preservation of renal structure and function following subtotal nephrectomyor streptozotocin-induced diabetes'
- AM. HEART J. Bd. 116, Nr. 6/1, 1988, Seiten 1591 - 1605; J.B. KOSTIS:'Angiotensin converting enzyme inhibitors. II. Clinical use'
- Z. KARDIOL. Bd. 77, Nr. S3, 1988, Seiten 73 - 88; D. KLAUS: 'Der Stellenwertvon Konversionsenzymhemmern in der Hypotoniebehandlung'
- Nieren- und Hochdruckkrankheiten, 21(10), S. 524-527, 1992
- Hypertension, 14(4), S. 429-435, 1990

## Beschreibung

Die vorliegende Erfindung betrifft die Prävention und Therapie einer Proteinurie durch kombinierte Gabe eines Angiotensin-Converting-Enzyme-Hemmers (ACE-Hemmer) und eines Calciumantagonisten.

ACE-Hemmer sind Verbindungen, welche die Umwandlung von Angiotensin I in das pressorisch wirksame Angiotensin II verhindern. Solche Verbindungen sind beispielsweise in den folgenden Patentanmeldungen oder Patenten beschrieben:
US-PS 4 350 633, US-PS 4 344 949,US-PS 4 294 832, US-PS 4 350 704, EP-A 31 741, EP-A 51 020, EP-A 49 658, EP-A 29 488, EP-A 46 953, EP-A 52 870, US-P 4 129 571, US-PS 4 154 960, US-PS 4 374 829, EP-A 79522, EP-A 79022, EP-A 51301, US-PS 4 454 292, US-PS 4 374 847, EP-A 72352, EP-A 84164, US-PS 4 470 972, EP-A-65301 und EP-A-52991. Ihre blutdrucksenkende Wirkung ist gut dokumentiert .

Calciumantagonisten sind solche Verbindungen, die den Einstrom von Calcium-Ionen in Zellen, insbesondere glatte Muskelzellen beeinflussen. Solche Verbindungen sowie ihre blutdrucksenkende Wirkung sind in einer Vielzahl von Publikationen und Patentanmeldungen niedergelegt.

Kombinationen von ACE-Hemmern und Calciumantagonisten und deren Verwendung bei der Behandlung des Bluthochdrucks sind beispielsweise bekannt aus EP-A-180 785 und EP-A-265 685.
Die blutdrucksenkende Wirkung nach kombinierter Gabe von ACE-Hemmern und Calciumantagonisten wird auch von D. Klaus [Z. Kardiol. Band 77 (Suppl. 3), 1988, S. 73-88] und J. B. Kostis [Am. Heart J. (1988) Band 116, Nr. 6/1, 1988, S. 1591-1605] beschrieben.

Die kombinierte Gabe von ACE-Hemmern und Calciumantagonistes bei der Behandlung einer Arzneimittelabhängigkeit ist aus EP-A 381 075 bekannt.

Eine Proteinurie tritt auf insbesondere bei Erkrankungen des Nierengewebes (Nephritis, Nephrose, Schrumpfniere) und bei Stauungsniere durch Herzinsuffizienz; außer den Albuminen gelangen auch die Globuline und andere Bluteiweißkörper in den Harn. Die höchsten Grade der Proteinurie finden sich bei Nephrose und Amyloidose. Eine konstante Proteinurie bei Diabetes mellitus weist auf die Entwicklung einer Glomerulosklerose (Kimmelstiel-Wilson' Syndrom) hin.

Eine Proteinurie-reduzierende Wirkung einiger ACE-Hemmer ist beschrieben [B. Kostis, Am. Heart J. (1988) Band 116, Nr. 6/1 S. 1591-1605; Z. Allgemeinmed. (1990) Band 66 (32), 924].

Studien an diabetischen Ratten zeigten, daß hingegen Calciumantagonisten eine Proteinurie nicht verbessern bzw. zu einer deutlichen Verschlechterung der Proteinurie führen. [B. Jackson et al., (1987), J. Cardiovasc. Pharmacology, 10 (Suppl 10), S167-S169].

Es wurde nun überraschenderweise gefunden, daß sich eine Kombination aus ACE-Hemmer und Calciumantagonist zur Präventation und Therapie einer Proteinurie eignet, wie diese typischerweise auftreten kann bei Diabetes mellitus und Nierenmassenverlust (Spenderniere), aber auch als Folgeerscheinung einer Glomerulosklerose infolge von Hyperperfusion der Glomeruli. Vorteilhaft sind oral wirksame ACE-Inhibitoren, wie z.B. Ramipril, Enalapril, Captopril, Alacepril, Benazepril, Ceranapril, Cilazapril, Delapril, Fosinopril, Imidapril, Libenzapril, Lisinopril, Moexipril, Moveltipril, Perindopril, Quinapril, Spirapril, Zofenopril, Trandolapril, BPL 36378, CS 622, FPL 63547, S 9650 und andere. Oral wirksame ACE-Inhibitoren sind beispielsweise in "Pharmaoology of Antihypertensive Therapeutics" (Eds. D. Ganten, P.J. Mutrow) Springer Verlag, Berlin 1990, S. 377-480 beschrieben.

Als ACE-Hemmer kommen insbesondere die folgenden Verbindungen der Formel I oder deren physiologisch verträglichen Salze in Betracht: 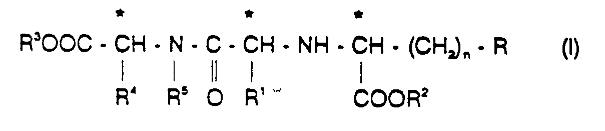
in welcher
n = 1 oder 2 ist,
R = Wasserstoff,
einen gegebenenfalls substituierten aliphatischen Rest mit 1 - 8 C-Atomen,
einen gegebenenfalls substituierten alicyclischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 14 C-Atomen,
einen gegebenenfalls substituierten alicyclisch-aliphatischen Rest mit 7 - 14 C-Atomen,
einen Rest OR^{a} oder SR^{a}, worin
R^{a} für einen gegebenenfalls substituierten aliphatischen Rest mit 1-4 C-Atomen, für einen gegebenenfalls substituierten aromatischen Rest mit 6 - 12 C-Atomen oder einen gegebenenfalls substituierten heteraromatischen Rest mit 5 - 12 Ringatomen steht.
R¹ Wasserstoff,
einen gegebenenfalls substituierten aliphatischen Rest mit 1 - 6 C-Atomen,
einen gegebenenfalls substituierten alicyclischen Rest mit 3 - 9 C-Atomen,
einen gegebenenfalls substituierten alicyclisch-aliphatischen Rest mit 4 - 13 C-Atomen,
einen gegebenenfalls substituierten aromatischen Rest mit 6 - 12 C-Atomen,
einen gegebenenfalls substituierten araliphatischen Rest mit 7 - 16 C-Atomen,
einen gegebenenfalls substituierten heteroaromatischen Rest mit 5 - 12 Ringatomen oder die erforderlichenfalls geschützte Seitenkette einer natürlichen vorkommenden α-Aminosäure bedeuten,
R² und R³ gleich oder verschieden sind und Wasserstoff,
einen gegebenenfalls substituierten aliphatischen Rest mit 1 - 6 C-Atomen,
einen gegebenenfalls substituierten alicyclischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 16 C-Atomen und
R⁴ und R⁵ zusammen mit den sie tragenden Atomen ein heterocyclisches, mono-, bi- oder tricyclische Ringsystem mit 3 bis 15 C-Atomen bilden, wobei als solche Ringsysteme insbesondere jene aus folgender Reihe in Betracht kommen:
Tetrahydroisochinolin (A); Decahydroisochinolin (B); Octahydroindol (C); Octahydrocyclopenta[b]pyrrol (D); 2-Azaspiro[4.5]decan (E); 2-Azaspiro[4.4]nonan (F); Spiro[(bicyclo[2.2.l]heptan)-2,3-pyrrolidin] (G); Spiro[bicyclo[2.2.2]octan)-2,3-pyrrolidin] (J); 2-Azatricyclo[4,3,0,1^{8,9}]decan (I); Decahydrocyclohepta[b]pyrrol (J); Octahydroisoindol (K); Octahydrocyclopenta[c]pyrrol (L); 2,3,3a,4,5,7a-Hexahydroindol (M); 2-Azabicyclo[3.1.0]hexan (N); Hexahydrocyclopenta[b]pyrrol (O); Pyrrolidin (P); Thiazolidin (Q); die alle gegebenenfalls substituiert sein können. Bevorzugt sind jedoch die unsubstituierten Systeme, welche die folgenden Strukturformeln aufweisen. 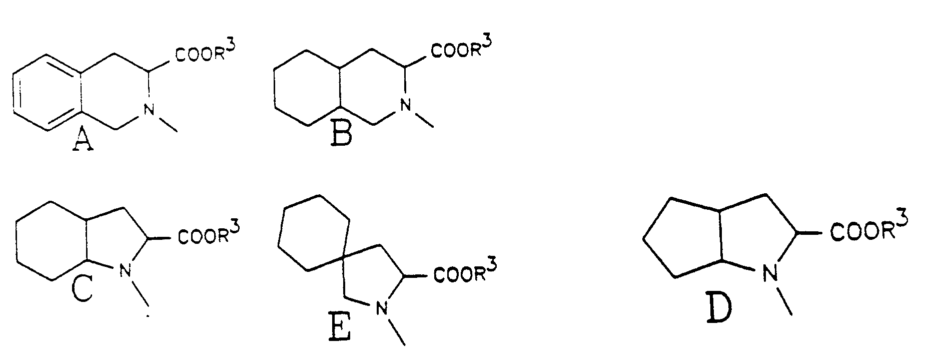 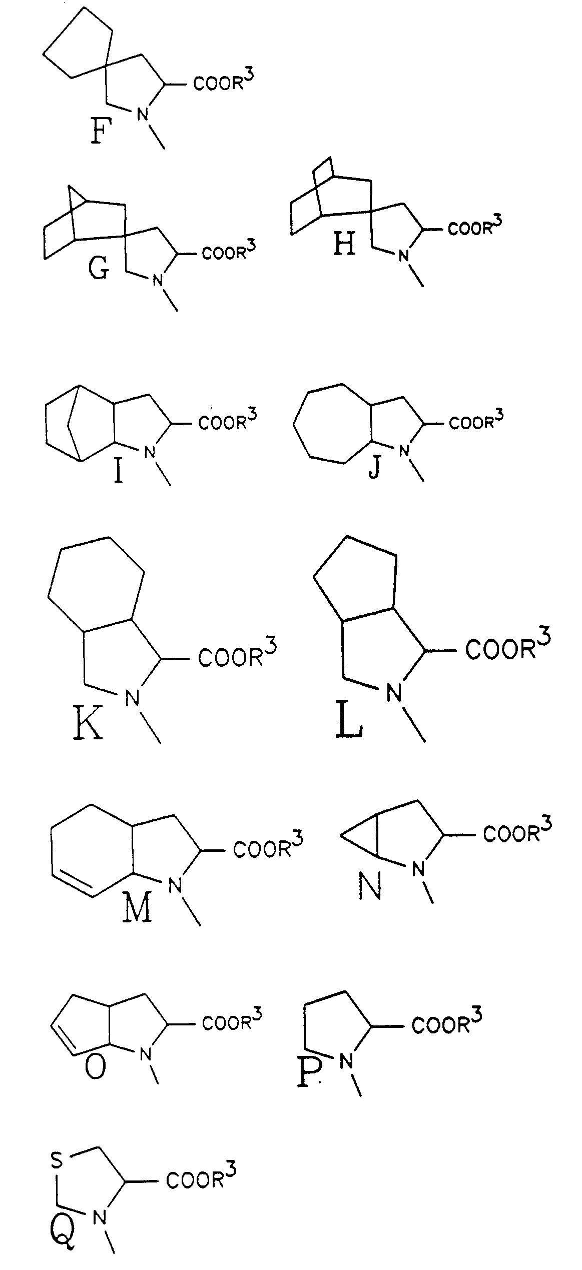
Bei den Verbindungen, die mehrere chirale Atome besitzen, kommen alle möglichen Diastereomere als Racemate oder Enantiomerere, oder Gemische verschiedener Diastereomere in Betracht. Bevorzugt ist die S-Konfiguration der mit einem Stern markierten C-Atome.

Bevorzugt sind ACE-Hemmer der Formel I, in der
n = 1 oder 2
R Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch (C₁C₄)-Alkyl, (C₁-C₄)-Alkoxy, Hydroxy, Halogen, Nitro, Amino, Aminoethyl, (C₁-C₄)-Alkylamino, mono-, di-oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 C-Atomen bzw. 8 - 10 Ringatomen,
wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-(C₁-C₄)-alkyl,
(C₁-C₄)-Alkanoylamino-(C₁-C₄)alkyl,
(C₇-C₁₃)-Aroylamino-(C₁-C₄)-alkyl,
(C₁-C₄)-Alkoxy-carbonylamino-(C₁-C₄)-alkyl,
(C₆-C₁₂)Aryl-(C₁-C₄)-alkoxycarbonylamino-(C₁-C₄)-alkyl,
(C₁-C₄)-Alkylamino-(C₁-C₄)-alkyl,
Di-(C₁-C₄)-alkylamino-(C₁-C₄)-alkyl,
Guanidino-(C₁-C₄)-alkyl,
Imidazolyl, Indolyl,
(C₁-C₄)-Alkylthio,
(C₁-C₄)-Alkylthio-(C₁-C₄)-alkyl,
(C₆-C₁₂)-Arylthio-(C₁-C₄)-alkyl,
das im Arylteil wie oben bei Aryl beschrieben, substituiert sein kann,
(C₆-C₁₂)-Aryl-(C₁-C₄)-alkylthio,
das im Arylteil wie oben bei Aryl beschrieben substituiert sein kann,
Carboxy-(C₁-C₄)-alkyl,
Carboxy, Carbamoyl,
Carbamoyl-(C₁-C₄)-alkyl,
(C₁-C₄)-Alkoxy-carbonyl-(C₁-C₄)-alkyl,
(C₆-C₁₂)-Aryloxy-(C₁-C₄)-alkyl,
das im Arylteil wie oben bei Aryl beschriebenen substituiert sein kann oder (C₆-C₁₂)-Aryl-(C₁-C₄)-alkoxy,
das im Arylteil wie oben bei Aryl beschrieben substituiert sein kann,
R¹ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
(C₃-C₉)-Cycloalkyl-(C₁-C₄)-alkyl,
(C₅-C₉)-Cycloalkenyl-(C₁-C₄)-alkyl,
gegebenenfalls teilhydriertes Aryl mit 6 - 12 C-Atomen, das wie oben bei R beschrieben substituiert sein kann,
(C₆-C₁₂)-Aryl-(C₁-C₄)-alkyl oder (C₇-C₁₃)-Aroyl-(C₁ oder C₂)alkyl,
die beide wie das vorstehende Aryl substituiert sein können mono- bzw. bicyclisches, gegebenenfalls teilhydriertes Heteroaryl mit 5 - 7 C-Atomen bzw. 8 - 10 Ringatomen, wovon 1 bis 2 Ringatome Schwefel- oder Sauerstoffatome und/oder 1 bis 4 Ringatome Stickstoffatome darstellen,
das wie vorstehende Aryl substituiert sein kann oder
die gegebenenfalls geschützte Seitenkette einer natürlich vorkommenden α-Aminosäure R¹-CH(NH₂)-COOH bedeuten,
R² und R³ gleich oder verschieden sind und Wasserstoff,
Alkyl mit 1 - 6 C-Atomen,
Alkenyl mit 2 - 6 C-Atomen,
Di-(C₁-C₄)-alkylamino-(C₁-C₄)-alkyl,
(C₁-C₅)-Alkanoyloxy-(C₁-C₄)-alkyl,
(C₁-C₆)-Alkoxy-carbonyloxy-(C₁-C₄)-alkyl,
(C₇-C₁₃)-Aryloxy-(C₁-C₄)-alkyl,
(C₆-C₁₂)-Aryloxycarbonyloxy-(C₁-C₄)-alkyl,
Aryl mit 6 - 12 C-Atomen,
(C₆-C₁₂)-Aryl-(C₁-C₄)-alkyl,
(C₃-C₉)-Cycloalkyl oder
(C₃-C₉)-Cycloalkyl-(C₁-C₄)-alkyl bedeuten und
R⁴ und R⁵ die oben angegebene Bedeutung haben,
ganz besonders bevorzugt sind Verbindungen der Formel I, in der
n = 1 oder 2 ist,
R (C₁-C₆)-Alkyl, (C₂-C₆)-Alkenyl, (C₃-C₉)-Cycloalkyl, Amino-(C₁-C₄)-alkyl, (C₂-C₅)-Acylamino-(C₁-C₄)-alkyl, (C₇-C₁₃)-Aroylamino-(C₁-C₄)-alkyl, (C₁-C₄)-Alkoxycarbonylamino-(C₁-C₄)-alkyl, (C₆-C₁₂)-Aryl-(C₁-C₄)-alkoxycarbonylamino-(C₁-C₄)-alkyl, (C₆-C₁₂)-Aryl, das durch (C₁-C₄)-Alkyl, (C₁-C₄)-Alkoxy, Hydroxy, Halogen, Nitro, Amino, (C₁-C₄)-Alkylamino, Di-(C₁C₄)-alkylamino und/oder Methylendioxy mono-, di- oder trisubstituiert sein kann, oder 3-Indolyl, insbesondere Methyl, Ethyl, Cyclohexyl, tert. Butoxycarbonylamino-(C₁-C₄)-alkyl, Benzoyloxycarbonylamino-(C₁-C₄)-alkyl oder Phenyl, das durch Phenyl, (C₁-C₂)-Alkyl, (C₁ oder C₂)-Alkoxy, Hydroxy, Fluor, Chlor, Brom, Amino, (C₁-C₄)-Alkylamino, Di-(C₁-C₄)alkylamino, Nitro und/oder Methylendioxy mono- oder disubstituiert oder im Falle von Methoxy, trisubstituiert sein kann, bedeutet,
R¹ Wasserstoff oder (C₁-C₆)-Alkyl, das gegebenenfalls durch Amino, (C₁-C₆)-Acylamino oder Benzoylamino substituiert sein kann, (C₂-C₆)-Alkenyl, (C₃-C₉)-Cycloalkyl, (C₅-C₉)-Cycloalkenyl, (C₃-C₇)-Cycloalkyl-(C₁-C₄)-alkyl, (C₆-C₁₂)-Aryl oder teilhydriertes Aryl, das jeweils durch (C₁-C₄)-Alkyl, (C₁ oder C₂)-Alkoxy oder Halogen substituiert sein kann, (C₆-C₁₂)-Aryl-(C₁ bis C₄)-alkyl oder (C₇-C₁₃)-Aroyl-(C₁-C₂)-alkyl, die beide wie vorstehend definiert im Arylrest substituiert sein können, ein mono- bzw. bicyclischer Heterocyclen-Rest mit 5 bis 7 C-Atomen bzw. 8 bis 10 Ringatome, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff. (C₁-C₄)-Alkyl, (C₂ oder C₃)-Alkenyl, die gegebenfalls geschrützte Seitenkette von Lysin, Benzyl, 4-Methoxybenzyl, 4-Ethoxybenzyl, Phenethyl, 4-Aminobutyl oder Benzoylmethyl bedeutet,
R² und R³ gleiche oder verschiedene Reste Wasserstoff, (C₁-C₆)-Alkyl, (C₂-C₆)-Alkenyl oder (C₆-C₁₂)-Aryl-(C₁-C₄)-alkyl, insbesondere aber Wasserstoff, (C₁-C₄)-Alkyl oder Benzyl bedeuten und
R⁴ und R⁵ die oben angegebene Bedeutung haben.

R⁴ und R⁵ stehen vorzugsweise zusammen für einen Rest der Formel 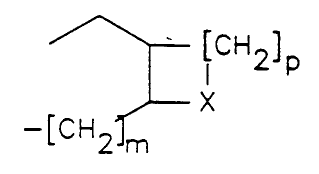
worin m = 0 oder 1, p = 0, 1 oder 2 und X = -CH₂-, -CH₂-CH₂- oder -CH=CH- bedeuten, wobei ein mit X gebildeter 6-Ring auch ein Benzolring sein kann.

Unter Aryl ist hier wie im folgenden vorzugsweise gegebenenfalls substituiertes Phenyl, Biphenyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 C-Atomen bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Phranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein. Natürlich vorkommende α-Aminosäuren sind z.B. in Houben-Weyl, Methoden der Organischen Chemie, Bd. XV/1 und XV/2 beschrieben.

Falls R¹ für eine Seitenkette einer geschützten natürlich vorkommenden α-Aminosäure steht, wie z.B. geschütztes Ser, Thr, Asp, Asn, Glu, Arg, Lys, Hyl, Cys, Orn, Cit, Tyr, Trp, His oder Hyp, sind als Schutzgruppen in der Peptidchemie üblichen Gruppen bevorzugt (vgl. Houben-Weyl, Bd. XV/1 und XV/2). Im Falle, daß R¹ die geschützte Lysin-Seitenkette bedeutet, werden die bekannten Amino-Schutzgruppen, Insbesondere aber Z, Boc oder (C₁-C₆)-Alkyl, insbesondere Methyl oder Ethyl in Frage.

Insbesondere bevorzugt sind die Verbindungen 2-[N-(1-S-Ethoxycarbonyl-3-phenylpropyl)-S-alanyl]-(1S,3S,5S)-2-azabicyclo[3.3.0]octan-3-carbonsäure (Ramipril), 1-[N-(1-S-Ethoxycarbonyl-3-phenylpropyl)-S-alanyl]-(2S,3aR,7aS)-octahydro[1H]indol-2-carbonsäure (Trandolapril), 2-(N-1-S-Ethoxycarbonyl-3-phenylpropyl)-S-alanyl]-1,2,3,4-tetrahydroisochinolin-3-S-carbonsäure (Quinapril), 1-[N-(1-S-Ethoxycarbonyl-3-phenylpropyl)-S-alanyl]-S-prolin (Enalapril), 1-[N-(1-S-Ethoxycarbonyl-3-phenylpropyl)-S-lysyl]-S-prolin (Lisinopril), 1[N-(1-S-Ethoxycarbonyl-n-butyl]-S-alanyl]-(2S,3aS,7aS)-octahydro[1H]indol-2-carbonsäure (Perindopril) sowie die entsprechenden Dicarbonsäuren. Vorteilhaft sind oral wirksame ACE-Inhibitoren, wie z.B. Ramipril, Enalapril, Captopril, Alacepril, Benazepril, Ceranapril, Cilazapril, Delapril, Fosinapril, Imdapril, Libenzapril, Lisinopril, Moexipril, Moveltipril, Perindopril, Quinapril, Spirapril, Zofenopril, Trandolapril, BPL 36378, CS 622, FPL 63547 und S 9650.

Die erfindungsgemäß verwendeten Verbindungen der Formel I kann man beispielsweise herstellen, indem man Verbindungen der Formel V mit Verbindungen der Formel VI umsetzt.

R³OOC - CH(R⁴) - NHR⁵ (V)

HOOC - CH(R¹) - NH - CH(COOR²) - (CH₂)ₙ - R (VI)

Die Umsetzung dieser Verbindungen kann beispielsweise in Analogie zu bekannten Peptidkupplungsverfahren in einem organischen Lösungsmittel wie DMF, CH₂Cl₂, DMA in Gegenwart von Kupplungshilfsmitteln, wie Carbodiimiden (z.B. Dicyclohexylcarbodiimid), Diphenylphosphorylazid, Alkanphosphonsäureanhydriden, Dialkylphosphinsäureanhydriden oder N,N-Succinimidylcarbonate in ein Lösungsmittel wie CH₃CN erfolgen. Aminogruppen in Verbindungen der Formel V können mit Teraethyldiphosphit aktiviert werden. Die Verbindungen der Formel VI können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76-136). Die Reaktion wird vorzugsweise zwischen -20°C und dem Siedepunkt des Reaktionsgemisches durchgeführt.

Ebenso lassen sich auch Verbindungen der Formel VII mit Verbindungen der Formel VIII unter Bildung von Verbindungen der Formel I umsetzen.

R³OOC - CH(R⁴) - N(R⁵ - CO - CH(R¹) - Y¹ (VII)

Y² - CH(COOR²) - (CH₂)ₙ - R (VIII)

worin entweder Y¹ für Amino und Y² für eine Abgangsgruppe oder Y¹ für eine Abgangsgruppe und Y² für Amino stehen. Geeignete Abgangsgruppen sind z.B. Cl, Br, I, Alkylsulfonyloxy oder Arylsufonyloxy.

Alkylierungen dieser Art führt man zweckmäßigerweise in Wasser oder einem organischen Lösungsmittel wie einem niedrigen aliphatischen Alkohol (wie Ethanol), Benzylalkohol, Acetonitril, Nitromethan oder Glycolether bei einer Temperatur zwischen -20°C und dem Siedepunkt des Reaktionsgemisches in Gegenwart einer Base wie einem Alkalimetallhydroxid oder einem organischen Amin durch.

Des weiteren lassen sich Verbindungen der Formel IX mit Verbindungen der Formel X kondensieren.

R³OOC - CH(R⁴) - N(R⁵) - CO - C(R¹) = Q¹ (IX)

Q² = C(COOR²) - (CH₂)ₙ - R (X)

worin entweder Q¹ für Amino + Wasserstoff und Q² für Oxo steht oder Q¹ für Oxo und Q² für Amino + Wasserstoff steht.

Die Kondensation wird zweckmäßigerweise in Wasser oder einem organischen Lösungsmittel, wie einem niederen aliphatischen Alkohol, bei einer Temperatur zwischen -20°C und dem Siedepunkt des Reaktionsgemisches in Gegenwart eines Reduktionsmittel, wie NaBH₃CN, durchgeführt, wobei Verbindungen der Formel I direkt erhalten werden. Man kann aber auch als Zwischenprodukte entstehenden Schiff'schen Basen oder Enamine gegebenenfalls nach vorheriger Isolierung unter Bildung von Verbindungen der Formel II reduzieren, beispielsweise durch Hydrierung in Gegenwart eines Übergangsmetallkatalysators.

Schließlich führt auch die Umsetzung von Verbindungen der Formel IX (Q¹ = H + NH₂) mit Verbindungen der Formel XI oder deren Umsetzung mit Verbindungen der Formeln XII und XIII zweckmäßig in Gegenwart einer Base, wie Natriumalkoholat, in einem organischen Lösungsmittel, wie einem niederen Alkohol, bei einer Temperatur zwischen -10°C und dem Siedepunkt des Reaktionsgemisches zu Verbindungen der Formel II (n=2),

R²OOC-CH=CH-COR (XI)

OCH-COOR² (XII)

R-CO-CH₃ (XIII)

wobei intermediär entstehende Schiff'sche Basen wie oben beschrieben reduziert und eine Carbonylgruppe reduktiv (beispielsweise mit einem komplexen Hydrid) in Methylen überführt werden.

In den obengenannten Formeln V - XIII sind R - R⁵ und n wie in Formel I definiert. Temporär zum Schutz nicht an der Reaktion beteiligter reaktiver Gruppen eingeführte Schutzgruppen werden nach beendeter Reaktion in an sich bekannter Weise abgespalten (vgl. Schröder, Lübke, loc cit., Seiten 1-75 und 246-270; Greene, "Protective Groups in Organic Synthesis", New York 1981).

Als Calciumantagonisten kommen die Verbindungen der Formel II 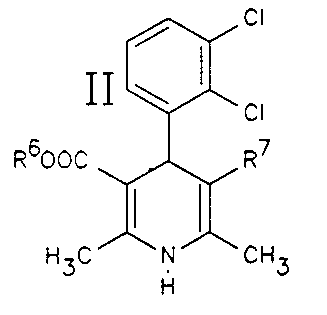
in Betracht, in welcher R⁶ Methyl, Ethyl oder Isopropyl und R⁷ Methoxycarbonyl, Ethoxycarbonyl oder 1,2,4-Oxadiazol-3-yl bedeuten, sowie deren physiologisch verträglichen Salze.

Weitere geeignete Calciumantagonisten sind 5-[(3,4-Dimethoxyphenylethyl)-methylamino]-2-(3,4-dimethoxyphenyl)-2-isopropylvaleronitril (Verapamil), 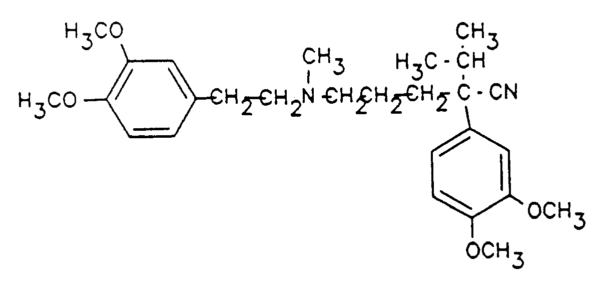
(2S-cis)-3-(Acetoxy)-5-[2-dimethylamino)-ethyl]-2,3-dihydroxy-2-(4-methoxyphenyl)-1,5-benzothiazepin-4(5H)-on (Diltiazem), 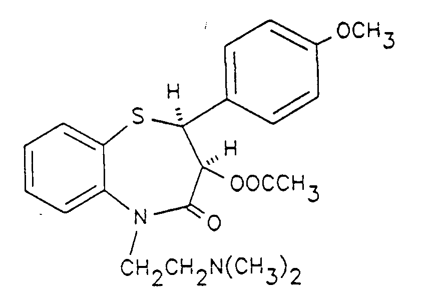
und 1,4-Dihydro-2,6-dimethyl-4-(2-nitrophenyl)-3,5-pyridindicarbonsäure-dimethylester (Nifedipin). 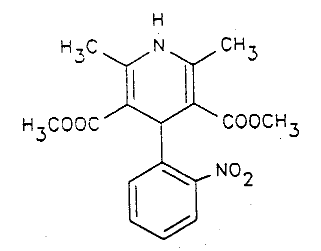
Neben den drei zuletzt genannten Calciumantagonisten sind insbesondere bevorzugt 4-(2,3-Dichlorphenyl)-2,6-dimethyl-3-methoxycarbonyl-5-ethoxycarbonyl-1,4-dihydropyridin (Felodipin) und 4-(2,3-Dichlorphenyl)-2,6-dimethyl-3-(1,2,4-oxadiazol-3-yl)-5-isopropoxycarbonyl-1,4-dihydropyridin sowie deren physiologisch verträglichen Salze mit Säuren.

Von ganz besonderen Interesse ist die Verwendung der folgenden Kombinationen:
Ramipril + Felodipin oder
Ramipril + 4(2,3-Dichlorphenyl)-2,6-dimethyl-3-(1,2,4-oxadiazol-5-yl)-5-isopropoxycarbonyl-1,4-dihydropyridin oder
Trandolapril + Felodipin oder
Trandolapril + 4-(2,3-Dichlorphenyl)-2,6-dimethyl-3-(1,2,4-oxadiazol-3-yl)-5-isopropoxycarbonyl-1,4-dihydropyridin oder
Trandolapril + Verapamil oder
Quinapril + Felodipin oder
Quinapril + 4-(2,3-Dichlorphenyl)-2,6-dimethyl-3-(1,2,4-oxadiazol-5-yl)-5-isopropoxycarbonyl-1,4-dihydropyridin
sowie jeweils die physiologisch verträglichen Salze der genannten Einzelkomponenten, soweit diese Salze bilden.

Neben der Anwendung der genannten Kombinationen betrifft die Erfindung auch die gleichzeitige, getrennte oder zeitlich abgestufte Anwendung von ACE-Hemmern und Calciumantagonisten bei der Behandlung der Proteinurie.

Die pharmazeutischen Zubereitungen können beispielsweise hergestellt werden, indem man entweder die Einzelkomponenten als Pulver intensiv mischt, oder indem man die Einzelkomponenten in einem geeigneten Lösungsmittel wie beispielsweise einem niederen Alkohol auflöst und das Lösungsmittel anschließend entfernt.

Das Verhältnis der Wirkstoffe in den erfindungsgemäßen Kombinationen und Zubereitungen beträgt vorzugsweise 1-15 Gewichtsteile ACE-Hemmer zu 15-1 Gewichtsteilen Calciumantagonist. Die erfindungsgemäßen Kombinationen und Zubereitungen enthalten insgesamt vorzugsweise 0,5-99,5 Gew.-%, insbesondere 4-96 Gew.-% dieser Wirkstoffe.

Bei der erfindungsgemäßen Anwendung bei Säugern, vorzugsweise beim Menschen, bewegen sich beispielsweise die Dosen eines ACE-Hemmers der o.g. Formel im Bereich von 0,05 bis 100 mg/kg/Tag und die eines Calciumantagonisten im Bereich von 0,05 bis 200 mg/kg/Tag.

Die pharmazeutischen Zubereitungen bzw. Erzeugnisse können parenteral oder oral verabreicht werden. Bevorzugt ist die orale Applikationsform.

Die pharmakologisch verwendbaren Kombinationen der vorliegenden Erfindung und ihre Salze können zur Herstellung von pharmazeutischen Präperaten verwendet werden, welche eine wirksame Menge der Aktivsubstanzen zusammen mit Trägerstoffen enthalten und die sich zur enteralen und parenteralen Verabreichung eignen. Vorzugsweise verwendet werden Tabletten oder Gelatinekapseln, welche die Wirkstoffe zusammen mit Verdünnungsmitteln, z.B. Lactose, Dextrose, Rohrzucker, Mannitol, Sorbitol, Cellulose und/oder Glycerin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganth, Methylcellulose, Natriumcarboxymethylcellulose und/oder Polyvinylpyrrolidon, und, falls benötigt, Farbstoff, Geschmacksstoffe und Süßmittel.

Injizierbare Lösungen sind vorzugsweise isotonische wäßrige Lösungen oder Suspensionen, die sterilisiert sein können.

Als Salze der obengenannten Verbindungen kommen, je nach sauerer oder basischer Natur dieser Verbindungen, Alkali- oder Erdalkalisalze oder Salze mit physiologisch verträglichen Aminen oder Salze mit anorganischen oder organischen Säuren wie z.B. HCl, HBr, H₂SO₄, Maleinsäure, Fumarsäure, Weinsäure, Citronensäure in Frage.

Präventation einer progressiven Proteinurie in Ratten mit Restniere

### 1. Methode

Männlichen ausgewachsenen Sprague-Dawley Ratten wurden zunächst 2/3 der rechten Niere durch Ligatur infarziert und im Abstand von einer Woche die kontralaterale Niere entfernt. Die Tiere erhielten unmittelbar danach über 8 Wochen die Medikation mit dem Futter. Aus dem Futterverbrauch ergab sich, daß Gruppe II (n:17) mit 58 mg/kg Felodipin (FE), Gruppe III (n:10) mit 1,6 mg/kg Ramipril (RA) und Gruppe IV (n:11) mit 41 mg/kg FE und 1,4 mg/kg RA behandelt wurden. Die Kontrollgruppe I erhielt Normalfutter. Der Urin wurde über jeweils 24 Stunden vor den Operationen und nach 1,3,5 und 7 Wochen gesammelt. Blutproben wurden aus dem retroorbitalen Plexus unter leichter Pentobarbitalnarkose jeweils am Tag zuvor und am Ende der Studie nach der Blutdruckmessung in der A. Karotis in finaler Pentobarbitainarkose gezogen.

### 2. Ergebnisse

Der mittlere arterielle Blutdruck betrug 160±7 mm Hg in der Kontrollgruppe 1, 122±3 mm Hg unter FE, 117±7 mm Hg unter RA und 108±4 mm Hg unter FE und RA. Die Proteinurie, die weniger als 20 mg/24 h vor der Operation betrug, vergrößerte sich in der Kontrollgruppe kontinuierlich bis auf 105±28 mg/24 h in der 7. Woche. Die Zunahme der Proteinurie verzögerte sich unter FE um 2 Wochen erreichte aber letzlich mit 114±28 mg/24 h ähnliche Werte. Mit 48±15 mg/24 h war die Proteinausscheidung unter RA deutlich geringer. Sie war nochmals auf nur 31±4 mg/24 h unter RA plus FE reduziert. Die Plasmakreatininspiegel verdoppelten sich nach der Operation in allen Gruppen von im Mittel 42 auf 73 mol/l, wurden aber danach durch die Medikation nicht weiter beeinflußt. Die Plasma-CE-Aktivität wurde unter RA mit und ohne FE um im Mittel 80% vermindert während FE allein keinen Einfluß hatte.

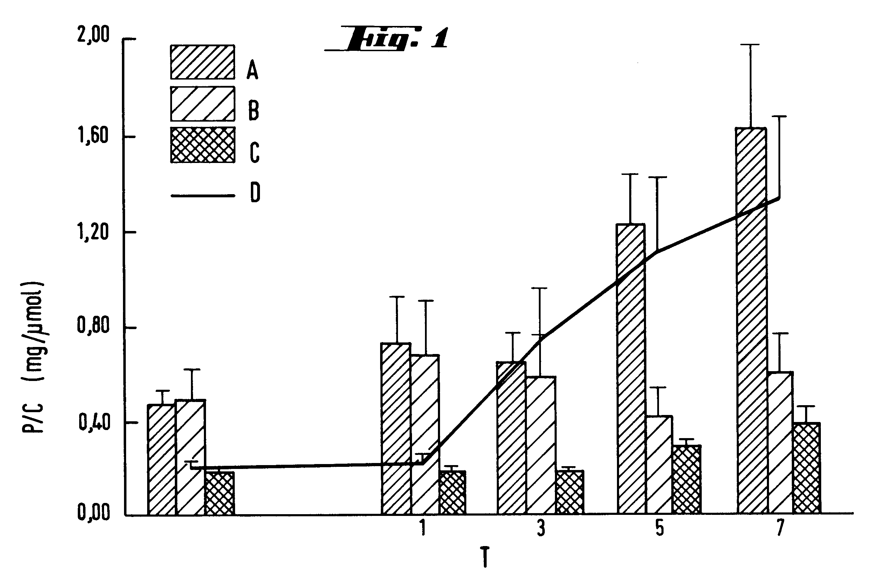

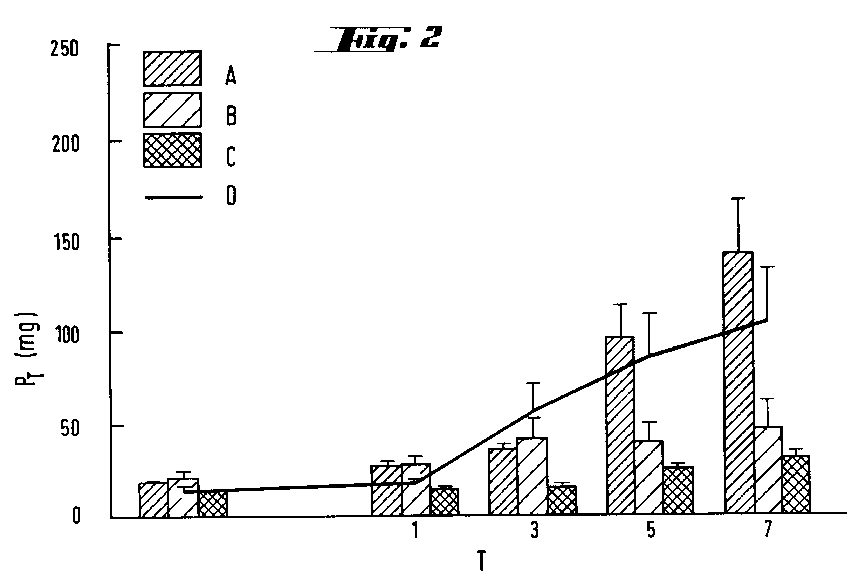

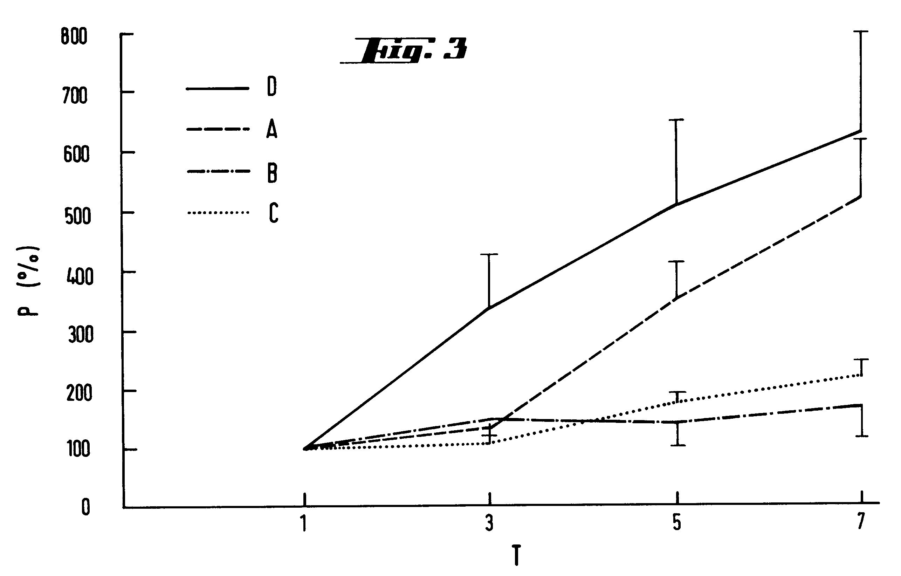

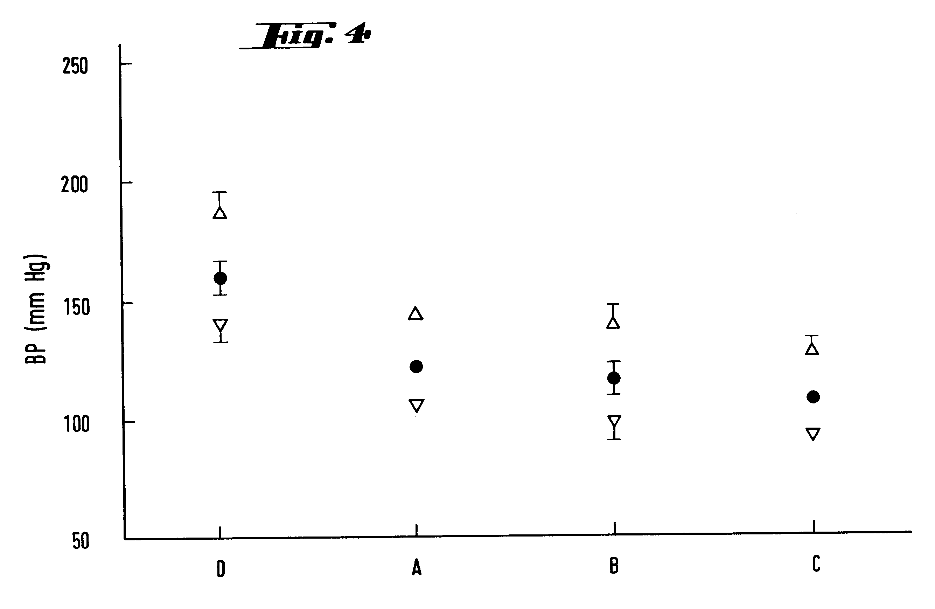

In Figur 1 und 2 ist T die Versuchsdauer in Wochen; die linken Werte ohne Zahl sind vor der Operation. Die in Figur 1-4 mit A bezeichneten Werte sind nach der Gabe von 58 mg/kg Felodipin, jene mit B nach Gabe von 1,6 mg/kg Ramipril, jene mit C nach Gabe einer Kombination von 1,4 mg Ramipril und 41 mg Felodipin gemessen. D stellt die Kontrolle dar.

Figur 1 zeigt das Verhältnis aus Protein-Ausscheidung und Kreatinin-Ausscheidung P/C in mg/µmol.

Figur 2 zeigt die Protein-Gesamtausscheidung P_{T} (mg) während 24 Stunden.

In Figur 3 ist die prozentuale Protein-Ausscheidung %P während 24 runden gegen die Versuchsdauer T aufgetragen.

Figur 4 zeigt den Blutdruck BP (mm Hg), wobei ● der mittlere Blutdruck, △ der systolische Blutdruck und ∇ der diastolische Blutdruck ist.

### 3. Diskussion

Ergebnisse von Versuchen mit Ratten belegen, daß Calciumantagonisten und ACE-Hemmer trotz gleicher Wirkung auf den systemischen Blutdruck in ihrer Wirkung auf die durch Verlust von Nephronen bedingte Verschlechterung der Nierenfunktion divergieren.

Die Untersuchung ergab weiter, daß eine Kontrolle des systemischen Blutdruckes mit einem Calciumantagonisten zwar den Beginn der Proteinurie verzögert, letztlich aber eher eine Beschleunigung des Nierenfunktionsverlustes bewirkt. Diese ungünstige Entwicklung konnte durch gleichzeitige Gabe eines ACE-Hemmers aufgehoben werden.

Die folgenden Beispiele dienen zur Erläuterung der vorliegenden Erfindung, ohne daß diese darauf beschränkt wäre:

### Beispiel 1:

### Herstellung eines oralen Kombinationspräparates aus Ramipril und Felodipin

1000 Tabletten, die 2 mg Ramipril und 6 mg Felodipin enthalten, werden wie folgt hergestellt:
Ramipril 2 g
Felodipin 6 g
Maisstärke 140 g
Gelatine 7,5 g
Mikrokristalline Cellulose 2,5 g
Magnesiumstearat 2,5 g
Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 2 mg Ramipril und 6 mg Felodipin enthält.

### Beispiel 2

### Herstellung eines oralen Kombinationspräparates aus Trandolapril und Verapamil

1000 Tabletten, die 3 mg Trandolapril und 50 mg Verapamil enthalten, werden wie folgt hergestellt:
Trandolapril 3 g
Verapamil 50 g
Maisstärke 130g
Gelatine 8,0 g
Mikrokristalline Cellulose 2,0 g
Magnesiumstearat 2,0 g
Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 3 mg Trandolapril und 50 mg Verapamil enthält.

### Beispiel 3:

### Herstellung eine oralen Kombinationspräparats aus Quinapril und Felodipin

1000 Tabletten, die 2,5 mg Quinapril und 6 mg Felodipin enthalten, werden wie folgt hergestellt:
Quinapril 2,5 g
Felodipin 5 g
Maisstärke 150 g
Gelatine 7,5 g
Mikrokristalline Cellulose 2,5 g
Magnesiumstearat 2,5 g
Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 2,5 mg Quinapril und 5 mg Felodipin enthält.

## Patentansprüche

1. Verwendung von ACE-Hemmer in Kombination mit Calciumantagonisten zur Herstellung eines Arzneimittels zur Prävention und Therapie einer Proteinurie wobei der ACE-Hemmer die Formel I aufweist, 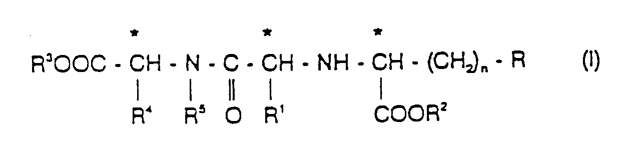 in welcher
n = 1 oder 2 ist,
R = Wasserstoff
einen gegebenenfalls substituierten aliphatischen Rest mit 1 - 8 C-Atomen,
einen gegebenenfalls substituierten alicyclischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 14 C-Atomen,
einen gegebenenfalls substituierten alicyclisch-aliphatischen Rest mit 7 - 14 C-Atomen,
einen Rest OR^{a} oder SR^{a}, worin
R^{a} für einen gegebenenfalls substituierten aliphatischen Rest mit 1 - 4 C-Atomen,
für einen gegebenenfalls substituierten aromatischen Rest mit 6 - 12 C-Atomen
oder einen gegebenenfalls substituierten heteroaromatischen Rest mit 5 - 12 Ringatomen steht,
R¹ Wasserstoff,
einen gegebenenfalls substituierten aliphatischen Rest mit 1 - 6 C-Atomen,
einen gegebenenfalls substituierten alicyclischen Rest mit 3 - 9 C-Atomen,
einen gegebenenfalls substituierten alicyclisch-aliphatischen Rest mit 4 - 13 C-Atomen,
einen gegebenenfalls substituierten aromatischen Rest mit 6 - 12 C-Atomen,
einen gegebenenfalls substituierten araliphatischen Rest mit 7 - 16 C-Atomen,
einen gegebenenfalls substituierten heteroaromatischen Rest mit 5 - 12 Ringatomen oder die erforderlichenfalls geschützte Seitenkette einer natürlich vorkommenden α-Aminosäure bedeuten,
R² und R³ gleich oder verschieden sind und Wasserstoff, einen gegebenenfalls substituierten aliphatischen Rest mit 1 - 6 C-Atomen,
einen gegebenenfalls substituierten alicyclischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 16 C-Atomen und
R⁴ und R⁵ zusammen mit den sie tragenden Atomen ein heterocyclisches mono-, bi- oder tricyclisches Ringsystem mit 3 bis 15 C-Atomen bilden,
oder dessen physiologisch verträgliches Salz ist.

2. Verwendung gemäß Anspruch 1 - , in welcher im ACE-Hemmer der Formel I R⁴ und R⁵ zusammen mit den diese tragenden Atomen ein Ringsystem aus der Reihe Tetrahydroisochinolin; Decahydroisochinolin; Octahydroindol; Octahydrocyclopenta[b]pyrrol; 2-Azaspiro[4.5]decan; 2-Azaspiro[4.4]nonan; Spiro[(bicyclo[2.2.1]heptan)-2,3'-pyrrolidin]; Spiro[(bicyclo[2.2.2] octan)-2,3'-pyrrolidin]; 2-Azatricyclo[4,3,0,1^{8.9})decan; Decahydrocyclohepta[b[pyrrol; Octahydroisoindol; Octahydrocyclopenta[c] pyrrol; 2,3,3a,4,5,7a-Hexahydroindol; 2-Azabicyclo[3.1.0]hexan; Hexahydrocyclopenta[b]pyrrol; Pyrrolidin und Thiazolidin, die alle gegebenenfalls substituiert sein können, bilden.

3. Verwendung gemäß einem der Ansprüche 1-2, in welcher im ACE-Hemmer der Formel I
n = 1 oder 2,
R = Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C-Atomen,
das durch (C₁-C₄)-Alkyl, (C₁-C₄)-Alkoxy, Hydroxy, Halogen, Nitro, Amino,
Aminomethyl, (C₁-C₄)-Alkylamino, Di-(C₁-C₄)-alkylamino, (C₁-C₄)-Alkanoylamino,
Methylendioxy, Cyano und/oder Sulfamoyl mono-, di- oder trisubstituiert sein kann Alkoxy mit 1 - 4 C-Atomen,
das wie oben bei Aryl beschrieben substituiert sein kann,
mono-bzw. bicyclisches Heteroaryloxy mit 5 - 7 C-Atomen bzw. 8 - 10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-(C₁-C₄)-alkyl,
(C₁-C₄)-Alkanoylamino-(C₁-C₄)-alkyl,
(C₇-C₁₃)-Aroylamino-(C₁-C₄)-alkyl,
(C₁-C₄)-Alkoxy-carbonylamino-(C₁-C₄)-alkyl,
(C₆-C₁₂)-Aryl-(C₁-C₄)-alkoxycarbonylamino-(C₁-C₄)-alkyl,
(C₆-C₁₂)-Aryl-(C₁-C₄)-alkylamino-(C₁-C₄)-alkyl,
(C₁-C₄)-Alkylamino-(C₁-C₄)-alkyl,
Di-(C₁-C₄)-alkylamino-(C₁-C₄)-alkyl,
Guanidino-(C₁-C₄)-alkyl,
Imidazolyl, Indolyl,
(C₁-C₄)-Alkylthio,
(C₁-C₄)-Alkylthio-(C₁-C₄)-alkyl,
(C₆-C₁₂)-Arylthio-(C₁-C₄)-alkyl,
das im Arylteil wie oben bei Aryl beschrieben, substituiert sein kann,
(C₆-C₁₂)-Aryl(C₁-C₄)-alkylthio,
das im Arylteil wie oben bei Aryl beschrieben substituiert sein kann,
Carboxy-(C₁-C₄)-alkyl,
Carboxy, Carbamoyl,
Carbamoyl-(C₁-C₄)-alkyl,
(C₁-C₄)-Alkoxy-carbonyl-(C₁-C₄)-alkyl,
(C₆-C₁₂)-Aryloxy-(C₁-C₄)-alkyl,
das im Arylteil wie oben bei Aryl beschrieben substituiert sein kann oder (C₆-C₁₂)-Aryl-(C₁-C₄)-alkoxy,
das im Arylteil wie oben bei Aryl beschrieben substituiert sein kann,
R¹ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
(C₃-C₉)-Cycloalkyl-(C₁-C₄)-alkyl,
(C₅-C₉)-Cycloalkenyl- (C₁-C₄)-alkyl,
gegebenenfalls teilhydriertes Aryl mit 6 - 12 C-Atomen, das wie oben bei R beschrieben substituiert sein kann,
(C₆-C₁₂)-Aryl(C₁-C₄)-alkyl oder (C₇-C₁₃)-Aroyl-(C₁ oder C₂)alkyl die beide wie das vorstehende Aryl substituiert sein können, mono- bzw. bicyclisches, gegebenenfalls teilhydriertes Heteroaryl mit 5-7 C-Atomen bzw. 8-10 Ringatomen, wovon 1 bis 2 Ringatome Schwefel- oder Sauerstoffatome und/oder 1 bis 4 Ringatome Stickstoffatome darstellen, das wie vorstehende Aryl substituiert sein kann
oder
die gegebenenfalls geschützte Seitenkette einer natürlich vorkommenden αAminosäure R¹-CH(NH₂)-COOH bedeuten,
R² und R³ gleich oder verschieden sind und Wasserstoff,
Alkyl mit 1 - 6 C-Atomen,
Alkenyl mit 2 - 6 C-Atomen,
Di-(C₁-C₄)-alkylamino-(C₁-C₄)-alkyl,
(C₁-C₅)-Alkanoyloxy-(C₁-C₄)-alkyl,
(C₁-C₆)-Alkoxy-carbonyloxy-(C₁-C₄)-alkyl,
(C₇-C₁₃)-Aryloxy-(C₁-C₄)-alkyl,
(C₆-C₁₂)-Aryloxycarbonyloxy-(C₁-C₄)-alkyl
Aryl mit 6 - 12 C-Atomen,
(C₆-C₁₂)-Aryl-(C₁-C₄)-alkyl,
(C₃-C₉)-Cycloalkyl oder
(C₃-C₉)-Cycloalkyl-(C₁-C₄)-alkyl bedeuten und
R⁴ und R⁵ die im Anspruch 1 oder 2 angegebene Bedeutung haben.

4. Verwendung gemäß einem der Ansprüche 1-3, in welcher im ACE-Hemmer der Formel I
n = 1 oder 2 ist,
R (C₁-C₆)-Alkyl, (C₂-C₆)-Alkenyl, (C₃-C₉)-Cycloalkyl, Amino-(C₁-C₄)-alkyl, (C₂-C₅)-Acylamino-(C₁-C₄)-alkyl, (C₇-C₁₃)-Aroylamino-(C₁-C₄)-alkyl, (C₁-C₄)-Alkoxycarbonylamino-(C₁-C₄)-alkyl, (C₆-C₁₂)-Aryl-(C₁-C₄)-alkoxycarbonylamino-(C₁-C₄)-alkyl, (C₆-C₁₂)-Aryl, das durch (C₁-C₄)-Alkyl, (C₁-C₄)Alkoxy, Hydroxy, Halogen, Nitro, Amino, (C₁-C₄)-Alkylamino, Di-(C₁-C₄)-alkylamino und/oder Methylendioxy mono-, di- oder trisubstituiert sein kann, oder 3-Indolyl, Insbesondere Methyl, Ethyl, Cyclohexyl, tert. Butoxycarbonylamino-(C₁-C₄)-alkyl, Benzoyloxycarbonylamino-(C₁-C₄)-alkyl oder Phenyl, das durch Phenyl, (C₁-C₂)-Alkyl, (C₁ oder C₂)-Alkoxy, Hydroxy, Fluor, Chlor, Brom, Amino, (C₁-C₄)-Alkylamino, DI-(C₁-C₄)alkylamino, Nitro und/oder Methylendioxy mono- oder disubstituiert oder Im Falle von Methoxy, trisubstituiert sein kann, bedeutet;
R¹ Wasserstoff oder (C₁-C₆)-Alkyl, das gegebenenfalls durch Amino, (C₁-C₆)-Acylamino oder Benzoylamino substituiert sein kann, (C₂-C₆)-Alkenyl, (C₃-C₉)-Cycloalkyl, (C₅-C₉)-Cycloalkenyl, (C₃-C₇)-Cycloalkyl-(C₁-C₄)-alkyl, (C₆-C₁₂)-Aryl oder teilhydriertes Aryl, das jeweils durch (C₁-C₄)-Alkyl, (C₁ oder C₂)-Alkoxy oder Halogen substituiert sein kann, (C₆-C₁₂)-Aryl-(C₁ bis C₄)-alkyl oder (C₇-C₁₃)-Aroyl-(C₁-C₂)-alkyl, die beide wie vorstehend definiert im Arylrest substituiert sein können, ein mono- bzw. bicyclischer Heterocyclen-Rest mit 5 bis 7 C-Atomen bzw. 8 bis 10 Ringatome, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff (C₁-C₃)-Alkyl, (C₂-C₃)-Alkenyl, die gegebenenfalls geschützte Seitenkette von Lysin, Benzyl, 4-Methoxybenzyl, 4-Ethoxybenzyl, Phenethyl, 4-Aminobutyl oder Benzoylmethyl bedeutet,
R² und R³ gleiche oder verschiedene Reste Wasserstoff, (C₁-C₆)-Alkyl, (C₂-C₆)-Alkenyl oder (C₆-C₁₂)-Aryl-(C₁-C₄)-alkyl, insbesondere aber Wasserstoff, (C₁-C₄)-Alkyl oder Benzyl bedeuten und
R⁴ und R⁵ die im Anspruch 1 angegebene Bedeutung haben.

5. Verwendung gemäß einem der Ansprüche 1-4, wobei der ACE-Hemmer 2-[N-(1-S-Ethoxycarbonyl-3-phenylpropyl)-S-alanyl]-(1S,3S,5S)-2-azabicyclo[3.3.0]octan-3-carbonsäure oder dessen physiologisch verträgliches Salz ist.

6. Verwendung gemäß einem der Ansprüche 1-4, wobei der ACE-Hemmer 1-[N-(1-S-Ethoxycarbonyl-3-phenylpropyl)-S-alanyl]-(2S,3aR,7aS)-octahydro[1H]indol-2-carbonsäure oder dessen physiologisch verträgliches Salz ist.

7. Verwendung gemäß einem der Ansprüche 1-4, wobei der ACE-Hemmer 2-[N-(1-S-Ethoxycarbonyl-3-phenylpropy)-S-alanyl]-1,2,3,4-tetrahydroisochinolin-3-S-carbonsäure oder dessen physiologisch verträgliches Salz ist.

8. Verwendung gemäß einem der Ansprüche 1-7, wobei der Calciumantagonist die Formel II aufweist 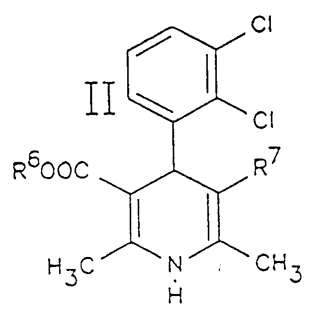 in welcher
R⁶ Methyl, Ethyl oder Isopropyl und
R⁷ Methoxycarbonyl, Ethoxycarbonyl oder 1,2,4-Oxadiazol-3-yl bedeuten, oder dessen physiologisch verträglichen Salze.

9. Verwendung gemäß einem der Ansprüche 1-7, wobei der Calciumantagonist eine Verbindung aus der Reihe Verapamil, Diltiazem und Nifedipin ist.

10. Verwendung gemäß einem der Ansprüche 1-7, wobei das Arzneimittel Ramipril + Felodipin oder Ramipril + 4-(2,3-Dichlorphenyl)-2,6-dimethyl-3-(1,2,4-oxadiazol-5-yl)-S-isopropoxycarbonyl-1,4-dihydropyridin oder
Trandolapril + Felodipin oder
Trandolapril + 4-(2,3-Dichlorphenyl)-2,6-dimethyl-3-(1,2,4-oxadiazol-3-yl)-5-isopropoxycarbonyl-1,4-dihydropyridin oder
Trandolapril + Verapamil oder
Quinapril + Felodipin oder
Quinapril + 4-(2,3-Dichlorphenyl)-2,6-dimethyl-3-(1,2,4-oxadiazol-5-yl)-5-isopropoxycarbonyl-1,4-dihydropyridin oder
jeweils das physiologisch verträgliche Salz der genannten Einzelkomponenten, soweit diese Salze bilden, enthält.

11. Verwendung gemäß Anspruch 10, wobei das Arzneimittel Trandolapril + Verapamil enthält.

12. Verwendung gemäß einem der Ansprüche 1-7, wobei der ACE-Hemmer eine Verbindung aus der Reihe Enalapril, Captopril, Alacepril, Benazepril, Ceranapril, Cilazapril, Delapril, Fosinopril, Imidapril, Libenzapril, Lisinopril, Moexipril, Moveltipril, Perindopril, Spirapril, Zofenopril, BPL 36378, C S622, FPL 63547 und S 9650 ist.

## Claims

1. The use of ACE inhibitors in combination with calcium antagonists for the production of a pharmaceutical for the prevention and therapy of proteinuria, where the ACE inhibitor has the formula I 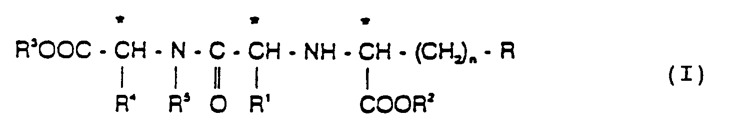 in which
n = 1 or 2,
R = hydrogen,
an optionally substituted aliphatic radical having 1 - 8 carbon atoms,
an optionally substituted alicyclic radical having 3 - 9 carbon atoms,
an optionally substituted aromatic radical having 6 - 12 carbon atoms,
an optionally substituted araliphatic radical having 7 - 14 carbon atoms,
an optionally substituted alicyclic-aliphatic radical having 7 - 14 carbon atoms,
a radical OR^{a} or SR^{a}, in which
R^{a} is an optionally substituted aliphatic radical having 1 - 4 carbon atoms, an optionally substituted aromatic radical having 6 - 12 carbon atoms or an optionally substituted heteroaromatic radical having 5 - 12 ring atoms,
R¹ is hydrogen,
an optionally substituted aliphatic radical having 1 - 6 carbon atoms,
an optionally substituted alicyclic radical having 3 - 9 carbon atoms,
an optionally substituted alicyclic-aliphatic radical having 4 - 13 carbon atoms,
an optionally substituted aromatic radical having 6 - 12 carbon atoms,
an optionally substituted araliphatic radical having 7 - 16 carbon atoms,
an optionally substituted heteroaromatic radical having 5 - 12 ring atoms or the side chain, which is protected if necessary, of a naturally occurring α-amino acid,
R² and R³ are identical or different and are hydrogen, an optionally substituted aliphatic radical having 1 - 6 carbon atoms,
an optionally substituted alicyclic radical having 3 - 9 carbon atoms,
an optionally substituted aromatic radical having 6 - 12 carbon atoms,
an optionally substituted araliphatic radical having 7 - 16 carbon atoms and
R⁴ and R⁵, together with the atoms carrying them,
form a heterocyclic mono-, bi- or tricyclic ring system having 3 to 15 carbon atoms,
or is its physiologically tolerable salt.

2. The use as claimed in claim 1, in which in the ACE inhibitor of the formula I, R⁴ and R⁵, together with the atoms carrying them, form a ring system from the series comprising tetrahydroisoquinoline; decahydroisoquinoline; octahydroindole; octahydrocyclopenta[b]pyrrole; 2-azaspiro[4.5]decane; 2-azaspiro[4.4]nonane; spiro[(bicyclo[2.2.1]heptane)-2,3'-pyrrolidine]; spiro[(bicyclo[2.2.2]octane)-2,3'-pyrrolidine];2-azatricyclo[4.3.0.1^{8,9}] decane; decahydrocyclohepta[b]pyrrole; octahydroisoindole; octahydrocyclopenta[c]pyrrole; 2,3,3a,4,5,7a-hexahydroindole; 2-azabicyclo[3.1.0]hexane; hexahydrocyclopenta[b]pyrrole; pyrrolidine and thiazolidine, which can all be optionally substituted.

3. The use as claimed in one of claims 1-2, in which in the ACE inhibitor of the formula I
n = 1 or 2,
R = hydrogen,
alkyl having 1 - 8 carbon atoms,
alkenyl having 2 - 6 carbon atoms,
cycloalkyl having 3 - 9 carbon atoms,
aryl having 6 - 12 carbon atoms,
which can be mono-, di- or trisubstituted by (C₁-C₄)-alkyl, (C₁-C₄)-alkoxy, hydroxyl, halogen, nitro, amino, aminomethyl, (C₁-C₄)-alkylamino, di-(C₁-C₄)-alkylamino, (C₁-C₄)-alkanoylamino, methylenedioxy, cyano and/or sulfamoyl,
alkoxy having 1 - 4 carbon atoms,
which can be substituted as described above for aryl,
mono- or bicyclic heteroaryloxy having 5 - 7 carbon atoms and 8 - 10 ring atoms, of which 1 to 2 ring atoms are sulfur or oxygen atoms and/or 1 to 4 ring atoms are nitrogen,
which can be substituted as described above for aryl,
amino-(C₁-C₄)-alkyl,
(C₁-C₄)-alkanoylamino-(C₁-C₄)-alkyl,
(C₇-C₁₃)-aroylamino-(C₁-C₄)-alkyl,
(C₁-C₄)-alkoxy-carbonylamino-(C₁-C₄)-alkyl,
(C₆-C₁₂)-aryl-(C₁-C₄)-alkoxycarbonylamino-(C₁-C₄)-alkyl,
(C₆-C₁₂)-aryl-(C₁-C₄)-alkylamino-(C₁-C₄)-alkyl,
(C₁-C₄)-alkylamino-(C₁-C₄)-alkyl,
di-(C₁-C₄)-alkylamino-(C₁-C₄)-alkyl,
guanidino-(C₁-C₄)-alkyl,
imidazolyl, indolyl,
(C₁-C₄)-alkylthio,
(C₁-C₄)-alkylthio-(C₁-C₄)-alkyl,
(C₆-C₁₂)-arylthio-(C₁-C₄)-alkyl,
which can be substituted in the aryl moiety as described above for aryl,
(C₆-C₁₂)-aryl-(C₁-C₄)-alkylthio,
which can be substituted in the aryl moiety as described above for aryl,
carboxy-(C₁-C₄)-alkyl,
carboxyl, carbamoyl,
carbamoyl-(C₁-C₄)-alkyl,
(C₁-C₄)-alkoxy-carbonyl-(C₁-C₄)-alkyl,
(C₆-C₁₂)-aryloxy-(C₁-C₄)-alkyl,
which can be substituted in the aryl moiety as described above for aryl or
(C₆-C₁₂)-aryl-(C₁-C₄)-alkoxy,
which can be substituted in the aryl moiety as described above for aryl,
R¹ is hydrogen,
alkyl having 1 - 6 carbon atoms,
alkenyl having 2 - 6 carbon atoms,
alkynyl having 2 - 6 carbon atoms,
cycloalkyl having 3 - 9 carbon atoms,
cycloalkenyl having 5 - 9 carbon atoms,
(C₃-C₉)-cycloalkyl-(C₁-C₄)-alkyl,
(C₅-C₉)-cycloalkenyl-(C₁-C₄)-alkyl,
optionally partially hydrogenated aryl having 6 - 12 carbon atoms, which can be substituted as described above for R,
(C₆-C₁₂)-aryl-(C₁-C₄)-alkyl or (C₇-C₁₃)-aroyl-(C₁ or C₂)-alkyl,
which can both be substituted like the above aryl, mono- or bicyclic, optionally partially hydrogenated heteroaryl having 5 - 7 carbon atoms and 8 - 10 ring atoms, of which 1 to 2 ring atoms are sulfur or oxygen atoms and/or 1 to 4 ring atoms are nitrogen atoms, which can be substituted like the above aryl or
the optionally protected side chain of a naturally occurring α-amino acid R¹-CH(NH₂)-COOH,
R² and R³ are identical or different and are hydrogen,
alkyl having 1 - 6 carbon atoms,
alkenyl having 2 - 6 carbon atoms,
di-(C₁-C₄)-alkylamino-(C₁-C₄)-alkyl,
(C₁-C₅)-alkanoyloxy-(C₁-C₄)-alkyl,
(C₁-C₆)-alkoxy-carbonyloxy-(C₁-C₄)-alkyl,
(C₇-C₁₃)-aryloxy-(C₁-C₄)-alkyl,
(C₆-C₁₂)-aryloxycarbonyloxy-(C₁-C₄)-alkyl,
aryl having 6 - 12 carbon atoms,
(C₆-C₁₂)-aryl-(C₁-C₄)-alkyl,
(C₃-C₉)-cycloalkyl or
(C₃-C₉)-cycloalkyl-(C₁-C₄)-alkyl and
R⁴ and R⁵ have the meaning indicated in claim 1.

4. The use as claimed in one of claims 1-3, in which in the ACE inhibitor of the formula I
n = 1 or 2,
R is (C₁-C₆)-alkyl, (C₂-C₆)-alkenyl, (C₃-C₉)-cycloalkyl, amino-(C₁-C₄)-alkyl, (C₂-C₅)-acylamino-(C₁-C₄)-alkyl, (C₇-C₁₃)-aroylamino-(C₁-C₄)-alkyl, (C₁-C₄)-alkoxycarbonylamino-(C₁-C₄)-alkyl, (C₆-C₁₂)-aryl-(C₁-C₄)-alkoxycarbonylamino-(C₁-C₄)-alkyl, (C₆-C₁₂)-aryl, which can be mono-, di- or trisubstituted by (C₁-C₄)-alkyl, (C₁-C₄)-alkoxy, hydroxyl, halogen, nitro, amino, (C₁-C₄)-alkylamino, di-(C₁-C₄)-alkylamino and/or methylenedioxy, or 3-indolyl, in particular methyl, ethyl, cyclohexyl, tert-butoxycarbonylamino-(C₁-C₄)-alkyl, benzoyloxycarbonylamino-(C₁-C₄)-alkyl or phenyl, which can be mono- or disubstituted or, in the case of methoxy, trisubstituted by phenyl, (C₁-C₂)-alkyl, (C₁ or C₂)-alkoxy, hydroxyl, fluorine, chlorine, bromine, amino, (C₁-C₄)-alkylamino, di-(C₁-C₄)-alkylamino, nitro and/or methylenedioxy;
R¹ is hydrogen or (C₁-C₆)-alkyl which can optionally be substituted by amino, (C₁-C₆)-acylamino or benzoylamino, (C₂-C₆)-alkenyl, (C₃-C₉)-cycloalkyl, (C₅-C₉)-cycloalkenyl, (C₃-C₇)-cycloalkyl-(C₁-C₄)-alkyl, (C₆-C₁₂)-aryl or partially hydrogenated aryl which in each case can be substituted by (C₁-C₄)-alkyl, (C₁ or C₂)-alkoxy or halogen, (C₆-C₁₂)-aryl-(C₁ to C₄)-alkyl or (C₇-C₁₃)-aroyl-(C₁-C₂)-alkyl, which can both be substituted in the aryl radical as defined above, a mono- or bicyclic heterocyclic radical having 5 to 7 carbon atoms and 8 to 10 ring atoms, of which 1 to 2 ring atoms are sulfur or oxygen atoms and/or 1 to 4 ring atoms are nitrogen atoms, or a side chain of a naturally occurring, optionally protected α-amino acid, but in particular hydrogen, (C₁-C₃)-alkyl, (C₂-C₃)-alkenyl, the optionally protected side chain of lysine, benzyl, 4-methoxybenzyl, 4-ethoxybenzyl, phenethyl, 4-aminobutyl or benzoylmethyl,
R² and R³ are identical or different hydrogen, (C₁-C₆)-alkyl, (C₂-C₆)-alkenyl or (C₆-C₁₂)-aryl-(C₁-C₄)-alkyl radicals, but in particular hydrogen, (C₁-C₄)-alkyl or benzyl and
R⁴ and R⁵ have the meaning indicated in claim 1.

5. The use as claimed in one of claims 1-4, where the ACE inhibitor is 2-[N-(1-S-ethoxycarbonyl-3-phenylpropyl)-S-alanyl]-(1S,3S,5S)-2-azabicyclo[3.3.0]octane-3-carboxylic acid or its physiologically tolerable salt.

6. The use as claimed in one of claims 1-4, where the ACE inhibitor is 1-[N-(1-S-ethoxycarbonyl-3-phenylpropyl)-S-alanyl]-(2S,3aR,7aS)-octahydro[1H]indole-2-carboxylic acid or its physiologically tolerable salt.

7. The use as claimed in one of claims 1-4, where the ACE inhibitor is 2-[N-(1-S-ethoxycarbonyl-3-phenylpropyl)-S-alanyl]-1,2,3,4-tetrahydroisoquinoline-3-S-carboxylic acid or its physiologically tolerable salt.

8. The use as claimed in one of claims 1-7, where the calcium antagonist has the formula II 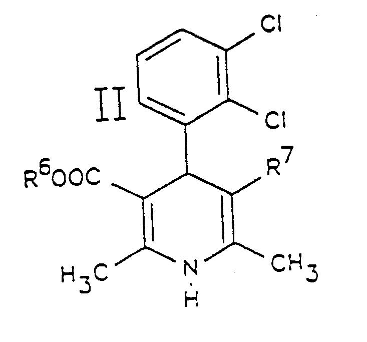 in which
R⁶ is methyl, ethyl or isopropyl and
R⁷ is methoxycarbonyl, ethoxycarbonyl or 1,2,4-oxadiazol-3-yl,
or its physiologically tolerable salts.

9. The use as claimed in one of claims 1-7, where the calcium antagonist is a compound from the series comprising verapamil, diltiazem and nifedipine.

10. The use as claimed in one of claims 1-7, where the pharmaceutical contains ramipril + felodipine or
ramipril + 4-(2,3-dichloro-phenyl)-2,6-dimethyl-3-(1,2,4-oxadiazol-5-yl)-5-isopropoxycarbonyl-1,4-dihydropyridine or
trandolapril + felodipine or
trandolapril + 4-(2,3-dichlorophenyl)-2,6-dimethyl-3-(1,2,4-oxadiazol-3-yl)-5-isopropoxycarbonyl-1,4-dihydropyridine or
trandolapril + verapamil or
quinapril + felodipine or
quinapril + 4-(2,3-dichlorophenyl)-2,6-dimethyl-3-(1,2,4-oxadiazol-5-yl)-5-isopropoxycarbonyl-1,4-dihydropyridine or
in each case the physiologically tolerable salt of said individual components, if these form salts.

11. The use as claimed in claim 10, where the pharmaceutical contains trandolapril + verapamil.

12. The use as claimed in one of claims 1-7, where the ACE inhibitor is a compound from the series comprising enalapril, captopril, alacepril, benazepril, ceranapril, cilazapril, delapril, fosinopril, imidapril, libenzapril, lisinopril, moexipril, moveltipril, perindopril, spirapril, zofenopril, BPL 36378, C S622, FPL 63547 and S 9650.

## Revendications

1. Utilisation d'inhibiteurs de l'enzyme de conversion de l'angiotensine (ACE) en asssociation avec des antagonistes du calcium, pour la fabrication d'un médicament destiné à la prévention et au traitement d'une protéinurie, l'inhibiteur d'ACE correspondant à la formule I 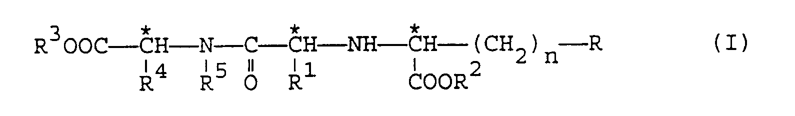 dans laquelle
n est 1 ou 2,
R représente un atome d'hydrogène
un radical aliphatique à 1-8 atomes de carbone éventuellement substitué,
un radical alicyclique à 3-9 atomes de carbone éventuellement substitué,
un radical aromatique à 6-12 atomes de carbone éventuellement substitué,
un radical araliphatique à 7-14 atomes de carbone éventuellement substitué,
un radical alicyclique-aliphatique à 7-14 atomes de carbone éventuellement substitué,
un radical OR^{a} ou SR^{a}, dans lequel
R^{a} représente un radical aliphatique à 1-4 atomes de carbone éventuellement substitué,
un radical aromatique à 6-12 atomes de carbone éventuellement substitué ou
un radical hétéroaromatique à 5-12 atomes de carbone formant le cycle, éventuellement substitué,
R¹ représente un atome d'hydrogène,
un radical aliphatique à 1-6 atomes de carbone éventuellement substitué,
un radical alicyclique à 3-9 atomes de carbone éventuellement substitué,
un radical alicyclique-aliphatique à 4-13 atomes de carbone éventuellement substitué,
un radical aromatique à 6-12 atomes de carbone éventuellement substitué,
un radical araliphatique à 7-16 atomes de carbone éventuellement substitué,
un radical hétéroaromatique à 5-12 atomes de carbone éventuellement substitué,
ou la chaîne latérale, protégée si nécessaire, d'un α-aminoacide existant dans la nature,
R² et R³ sont identiques ou différents et représentent un atome d'hydrogène ou un radical aliphatique à 1-6 atomes de carbone éventuellement substitué,
un radical alicyclique à 3-9 atomes de carbone éventuellement substitué,
un radical aromatique à 6-12 atomes de carbone éventuellement substitué,
un radical araliphatique à 7-16 atomes de carbone éventuellement substitué, et
R⁴ et R⁵ forment ensemble, avec les atomes qui les portent, un système cyclique hétérocyclique mono-, bi- ou tricyclique ayant de 3 à 15 atomes de carbone,
ou est un sel physiologiquement acceptable d'un tel composé.

2. Utilisation selon la revendication 1, dans laquelle, dans l'inhibiteur d'ACE de formule 1, R⁴ et R⁵ forment ensemble, avec les atomes qui les portent, un système cyclique de la série tétrahydroisoquinoléine, décahydroisoquinoléine, octahydroindole, octahydrocyclopenta[b]pyrrole, 2-azaspiro[4.5]décane, 2-azaspiro[4.4]nonane, spiro[(bicyclo[2.2.1]heptane)-2,3'-pyrrolidine], spiro[(bicyclo[2.2.2]octane)-2,3'-pyrrolidine], 2-azatricyclo[4,3,0,1^{8.9}]décane, décahydrocyclohepta[b]pyrrole, octahydroisoindole, octahydrocyclopenta[c]pyrrole, 2,3,3a,4,5,7a-hexahydroindole, 2-azabicyclo[3.1.0]hexane, hexahydrocyclopenta[b]pyrrole, pyrrolidine et thiazolidine, qui peuvent tous être éventuellement substitués.

3. Utilisation selon la revendication 1 ou 2, dans laquelle, dans l'inhibiteur d'ACE de formule I,
n est 1 ou 2,
R représente un atome d'hydrogène,
un radical alkyle ayant de 1 à 8 atomes de carbone,
alcényle ayant de 2 à 6 atomes de carbone,
cycloalkyle ayant de 3 à 9 atomes de carbone,
aryle ayant de 6 à 12 atomes de carbone,
qui peut être mono-, di- ou trisubstitué par un ou des atomes d'halogène ou groupes alkyle en C₁-C₄, alcoxy en C₁-C₄, hydroxy, nitro, amino, aminométhyle, alkyl(C₁-C₄)amino, dialkyl(C₁-C₄)amino, alcanoyl(C₁-C₄)amino, méthylènedioxy, cyano et/ou sulfamoyle,
alcoxy ayant de 1 à 4 atomes de carbone, qui peut être substitué comme décrit pour le radical aryle, hétéroaryloxy mono- ou bicyclique ayant 5-7 atomes de carbone ou, respectivement, 8-10 atomes formant le ou les cycles, dont 1 ou 2 atomes formant le ou les cycles représentent des atomes d'oxygène ou de soufre et/ou 1 à 4 atomes formant le ou les cycles représentent des atomes d'azote,
qui peut être substitué comme décrit pour le radical aryle,
aminoalkyle(C₁-C₄),
alcanoyl(C₁-C₄)amino-alkyle(C₁-C₄),
aroyl(C₇-C₁₃)amino-alkyle(C₁-C₄),
alcoxy(C₁-C₄)-carbonylamino-alkyle(C₁-C₄),
aryl(C₆-C₁₂)-alcoxy(C₁-C₄)carbonylamino-alkyle(C₁-C₄),
aryl(C₆-C₁₂)-alkyl(C₁-C₄)amino-alkyle(C₁-C₄),
alkyl(C₁-C₄)amino-alkyle(C₁-C₄),
dialkyl(C₁-C₄)amino-alkyle(C₁-C₄),
guanidino-alkyle(C₁-C₄),
imidazolyle, indolyle,
alkyl(C₁-C₄)thio,
alkyl(C₁-C₄)thio-alkyle(C₁-C₄),
aryl(C₆-C₁₂)thio-alkyle(C₁-C₄),
qui peut être substitué sur le fragment aryle comme décrit pour le radical aryle,
aryl(C₆-C₁₂)-alkyl(C₁-C₄)thio,
qui peut être substitué sur le fragment aryle comme décrit pour le radical aryle,
carboxy-alkyle(C₁-C₄),
carboxy, carbamoyle,
carbamoyl-alkyle(C₁-C₄),
alcoxy(C₁-C₄)-carbonyl-alkyle(C₁-C₄),
aryloxy(C₆-C₁₂)-alkyle(C₁-C₄),
qui peut être substitué sur le fragment aryle comme décrit pour le radical aryle, ou
aryl(C₆-C₁₂)-alcoxy(C₁-C₄),
qui peut être substitué sur le fragment aryle comme décrit pour le radical aryle,
R¹ représente un atome d'hydrogène,
un radical alkyle ayant de 1 à 6 atomes de carbone,
alcényle ayant de 2 à 6 atomes de carbone,
alcynyle ayant de 2 à 6 atomes de carbone,
cycloalkyle ayant de 3 à 9 atomes de carbone,
cycloalcényle ayant de 5 à 9 atomes de carbone,
cycloalkyl(C₃-C₉)-alkyle(C₁-C₄),
cycloalcényl(C₅-C₉)-alkyle(C₁-C₄),
un radical aryle à 6-12 atomes de carbone, éventuellement partiellement hydrogéné, qui peut être substitué comme décrit plus haut pour R,
aryl(C₆-C₁₂)-alkyle(C₁-C₄) ou aroyl(C₇-C₁₃)-alkyle(C₁ ou C₂),
qui peuvent être l'un et l'autre substitués comme le radical aryle précédent,
un radical hétéroaryle mono- ou bicyclique, éventuellement partiellement hydrogéné, ayant 5-7 atomes de carbone ou,
respectivement, 8-10 atomes formant le ou les cycles, dont 1 ou 2 atomes formant le ou les cycles représentent des atomes d'oxygène ou de soufre et/ou 1-4 atomes formant le ou les cycles représentent des atomes d'azote, qui peut être substitué comme le radical aryle précédent, ou
la chaîne latérale éventuellement protégée d'un α-aminoacide R¹-CH(NH₂)-COOH existant dans la nature,
R² et R³ sont identiques ou différents et représentent un atome d'hydrogène,
un radical alkyle ayant de 1 à 6 atomes de carbone,
alcényle ayant de 2 à 6 atomes de carbone,
dialkyl(C₁-C₄)amino-alkyle(C₁-C₄),
alcanoyloxy(C₁-C₆)-alkyle(C₁-C₄),
alcoxy(C₁-C₆)-carbonyloxy-alkyle(C₁-C₄),
aryloxy(C₇-C₁₃)-alkyle(C₁-C₄),
aryloxy(C₆-C₁₂)-carbonyloxy-alkyle(C₁-C₄),
un radical aryle ayant de 6 à 12 atomes de carbone,
aryl(C₆-C₁₂)-alkyle(C₁-C₄),
cycloalkyle en C₃-C₉ ou
cycloalkyl(C₃-C₉)-alkyle(C₁-C₄) et
R⁴ et R⁵ ont les significations données dans la revendication 1 ou 2.

4. Utilisation selon l'un des revendication 1 à 3, dans laquelle, dans l'inhibiteur d'ACE De formule I
n est 1 ou 2,
R représente un radical alkyle en C₁-C₆, alcényle en C₂-C₆, cycloalkyle en C₃-C₉, amino-alkyle(C₁-C₄), acyl(C₂-C₃)amino-alkyle(C₁-C₄), aroyl(C₇-C₁₃)amino-alkyle-(C₁-C₄), alcoxy(C₁-C₄)-carbonylamino-alkyle(C₁-C₄), aryl(C₆-C₁₂)-alcoxy(C₁-C₄)-carbonylamino-alkyle(C₁-C₄), un radical aryle en C₆-C₁₂ qui peut être mono-, di- ou trisubstitué par un ou des atomes d'halogène ou groupes alkyle en C₁-C₄, alcoxy en C₁-C₄, hydroxy, nitro, amino, alkyl(C₁-C₄)amino, dialkyl(C₁-C₄)amino et/ou méthylènedioxy, ou un radical 3-indolyle, en particulier un radical méthyle, éthyle, cyclohexyle, tert-butoxycarbonylamino-alkyle(C₁-C₄), benzoyloxycarbonylamino-alkyle(C₁-C₄) ou phényle qui peut être mono- ou disubstitué ou, dans le cas du radical méthoxy, trisubstitué par un ou des atomes de fluor, chlore ou brome ou groupes phényle, alkyle en C₁-C₂, alcoxy en C₁ ou C₂, hydroxy, amino, alkyl- (C₁-C₄)amino, dialkyl(C₁-C₄)amino, nitro et/ou méthylènedioxy;
R¹ représente un atome d'hydrogène ou un radical alkyle en C₁-C₆ qui peut éventuellement être substitué par un groupe amino, acyl(C₁-C₆)amino ou benzoylamino, un radical alcényle en C₂-C₆, cycloalkyle en C₃-C₉, cycloalcényle en C₅-C₉, cycloalkyl(C₃-C₇)-alkyle(C₁-C₄), aryle en C₆-C₁₂ ou aryle en C₆-C₁₂ partiellement hydrogéné, qui peut dans chaque cas être substitué par un groupe alkyle en C₁-C₄, alcoxy en C₁ ou C₂ ou par un atome d'halogène, un radical aryl(C₆-C₁₂)-alkyle-(C₁-C₄) ou aroyl(C₇-C₁₃)-alkyle(C₁-C₂), qui peuvent l'un et l'autre être substitués sur le fragment aryle comme défini précédemment, un radical hétérocyclique mono- ou bicyclique ayant de 5 à 7 atomes de carbone ou, respectivement, de 8 à 10 atomes formant le ou les cycles, dont 1 ou 2 atomes formant le ou les cycles représentent des atomes d'oxygène ou de soufre et/ou 1 à 4 atomes formant le ou les cycles représentent des atomes d'azote, ou une chaîne latérale d'un α-aminoacide existant dans la nature, éventuellement protégé, mais en particulier un atome d'hydrogène ou un radical alkyle en C₁-C₃, alcényle en C₂-C₃, la chaîne latérale éventuellement protégée de la lysine, le radical benzyle, 4-méthoxybenzyle, 4-éthoxybenzyle, phénéthyle, 4-aminobutyle ou benzoylméthyle;
R² et R³ sont identiques ou différents et représentent un atome d'hydrogène ou un radical alkyle en C₁-C₆, alcényle en C₂-C₆ ou aryl(C₆-C₁₂)-alkyle(C₁-C₄), mais en particulier un atome d'hydrogène ou un radical alkyle en C₁-C₄ ou benzyle, et
R⁴ et R⁵ ont les significations données dans la revendication 1.

5. Utilisation selon l'une des revendications 1 à 4, dans laquelle l'inhibiteur d'ACE est l'acide 2-[N-(1-S-éthoxycarbonyl-3-phénylpropyl)-S-alanyl]-(1S,3S,5S)-2-aza bicyclo[3.3.0]octane-3-carboxylique ou un sel physiologiquement acceptable de celui-ci.

6. Utilisation selon l'une des revendications 1 à 4, dans laquelle l'inhibiteur d'ACE est l'acide 1-[N-(1-S-éthoxycarbonyl-3-phénylpropyl)-S-alanyl]-(2S,3aR,7aS)-octahydro[1H]indol-3-carboxylique ou un sel physiologiquement acceptable de celui-ci.

7. Utilisation selon l'une des revendications 1 à 4, dans laquelle l'inhibiteur d'ACE est l'acide 2-[N-(1-S-éthoxycarbonyl-3-phénylpropyl)-S-alanyl]-1,2,3,4-tétrahydroisoquinoléine-3-S-carboxylique ou un sel physiologiquement acceptable de celui-ci.

8. Utilisation selon l'une des revendications 1 à 7, dans laquelle l'antagoniste du calcium correspond à la formule II 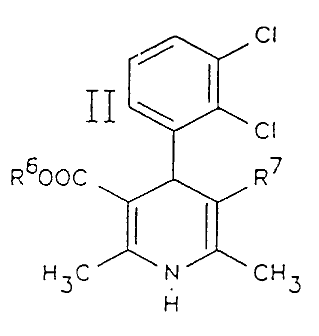 dans laquelle
R⁶ représente le groupe méthyle, éthyle ou isopropyle et
R⁷ représente le groupe méthoxycarbonyle, éthoxycarbonyle ou 1,2,4-oxadiazol-3-yle,
ou est un des sels physiologiquement acceptables d'un tel composé.

9. Utilisation selon l'une des revendications 1 à 7, dans laquelle l'antagoniste du calcium est un composé choisi parmi le vérapamil, le diltiazem et la nifédipine.

10. Utilisation selon l'une des revendications 1 à 7, dans laquelle le médicament contient:
ramipril + félodipine ou ramipril + 4-(2,3-dichlorophényl)-2,6-diméthyl-3-(1,2,4-oxadiazol-5-yl)-S-isopropoxycarbonyl-1,4-dihydropyridine ou
trandolapril + félodipine ou
trandolapril + 4-(2,3-dichlorophényl)-2,6-diméthyl-3-(1,2,4-oxadiazol-3-yl)-5-isopropoxycarbonyl-1,4-dihydropyridine ou
trandolapril + vérapamil ou
quinapril + félodipine ou
quinapril + 4-(2,3-dichlorophényl)-2,6-diméthyl-3-(1,2,4-oxadiazol-5-yl)-5-isopropoxycarbonyl-1,4-dihydropyridine ou
dans chaque cas le sel physiologiquement acceptable des composants individuels cités, dans la mesure ou ceux-ci forment des sels.

11. Utilisation selon la revendication 10, dans laquelle le médicament contient du trandolapril + vérapamil.

12. Utilisation selon l'une des revendications 1 à 7, dans laquelle l'inhibiteur d'ACE est un composé choisi parmi les suivants: énalapril, captopril, alacépril, bénazépril, céranapril, cilazapril, délapril, fosinopril, imidapril, libenzapril, lisinopril, moexipril, moveltipril, perindopril, spirapril, zofénopril, BPL 36378, C S622, FPL 63547 et S 9650.
